(19) Europäisches Patentamt
European Patent Office
Office européen des brevets

(11) Veröffentlichungsnummer: **0 187 245**
**B1**

(12) # EUROPÄISCHE PATENTSCHRIFT

(45) Veröffentlichungstag der Patentschrift:
29.06.88

(51) Int. Cl.⁴: **G 21 K 1/04,** A 61 B 6/06

(21) Anmeldenummer: 85115076.3

(22) Anmeldetag: 27.11.85

(54) Primärstrahlenblende für Röntgendiagnostikgeräte.

(30) Priorität: 11.12.84 DE 8436281 U

(43) Veröffentlichungstag der Anmeldung:
16.07.86 Patentblatt 86/29

(45) Bekanntmachung des Hinweises auf die Patenterteilung:
29.06.88 Patentblatt 88/26

(84) Benannte Vertragsstaaten:
DE FR

(56) Entgegenhaltungen:
EP-A-0 068 202
DE-A-2 842 659
DE-A-3 236 082
GB-A-1 258 028
US-A-4 203 037
US-A-4 315 146

(73) Patentinhaber: Siemens Aktiengesellschaft Berlin und München, Wittelsbacherplatz 2, D-8000 München 2 (DE)

(72) Erfinder: Telorack, Gerhard, Röthenäckerstrasse 10, D-8521 Aurachtal (DE)

## Beschreibung

Die Erfindung betrifft eine Primärstrahlenblende gemäß dem Oberbegriff des Patentanspruches.

Es sind Primärstrahlenblenden bekannt, bei denen in zwei parallelen Ebenen jeweils zwei Blendenplattenpaare gegenläufig zueinander verstellbar sind, so daß eine rechteckförmige Einblendung des Röntgenstrahlenbündels möglich ist. Die Verstellung übereinanderliegender Blendenplattenpaare muß dabei in der Weise erfolgen, daß nur die das Strahlungsfeld definierende Öffnung freigelassen wird.

In der DE-A-3 236 082 ist eine Primärstrahlenblende gemäß dem Oberbegriff des Patentanspruches beschrieben, bei der sichergestellt ist, daß sich die parallel liegenden Blendenplatten auf jeder Blendenseite in jeder Blendenstellung überdecken. Hierzu sind zwei Seilzüge sowie eine Vielzahl von Rollen vorgesehen, über die die Seilzüge geführt sind. Ein Motor treibt einen Seilzug an und der zweite Seilzug mit den zugeordneten Blendenplatten wird über Mitnehmer mitgenommen.

Der Erfindung liegt die Aufgabe zugrunde, eine Primärstrahlenblende der eingangs genannten Art hinsichtlich ihres Aufbaus gegenüber dem Stand der Technik zu vereinfachen, wobei eine störungssichere Konstruktion geschaffen werden soll, bei der das Klemmen der Seilzüge verhindert ist.

Diese Aufgabe ist erfindungsgemäß durch die im kennzeichnenden Teil des Patentanspruches angegebene Ausbildung gelöst. Bei der erfindungsgemäßen Primärstrahlenblende sind für die Erzielung unterschiedlicher Stellwege der Blendenplatten in den einzelnen Ebenen lediglich mindestens zwei Rollen mit unterschiedlichem Durchmesser vorhanden, die auf einer gemeinsamen Achse sitzen. Aufgrund der unterschiedlichen Durchmesser wird dabei das angestrebte Ziel erreicht.

Die Erfindung ist nachfolgend anhand eines in der Zeichnung dargestellten Ausführungsbeispieles näher erläutert. Es zeigen:

Fig. 1 eine Primärstrahlenblende zur Erläuterung des Erfindungsgedankens, und

Fig. 2 eine Einzelheit der Primärstrahlenblende gemäß Fig. 1.

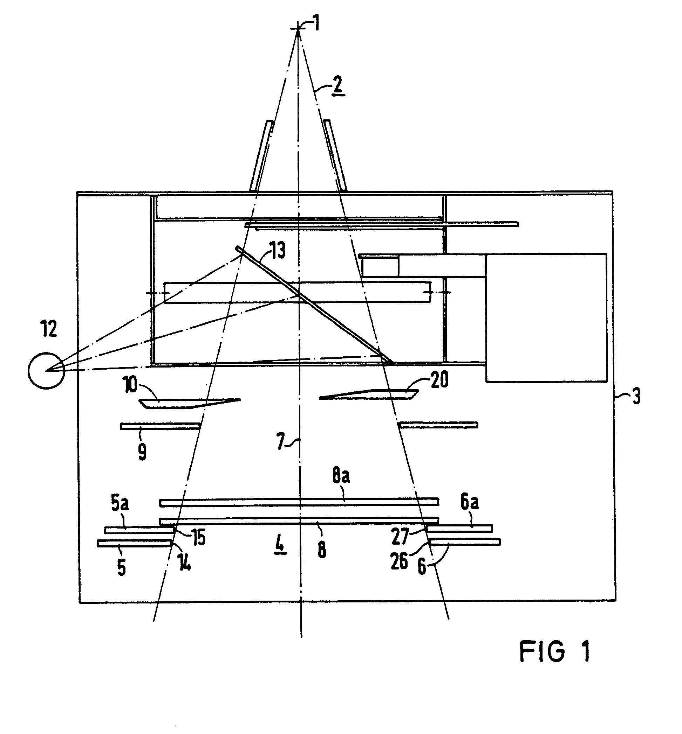

In der Fig. 1 ist der Fokus 1 einer Röntgenröhre dargestellt, dessen Röntgenstrahlenbündel 2 durch eine Primärstrahlenblende 3 pyramidenförmig eingegrenzt wird. Hierzu ist eine Rechteckblende 4 vorhanden, die in zwei parallelen Ebenen zwei Blendenplattenpaare 5, 6 bzw. 5a, 6a aufweist, deren Blendenplatten gegenläufig zueinander bewegbar sind. Dadurch ist die Breite des Röntgenstrahlenbündels 2 einstellbar. Die Einstellung des Röntgenstrahlenbündels 2 in der anderen Dimension erfolgt durch zwei weitere in parallelen Ebenen liegende Blendenplattenpaare, von denen in der Fig. 1 nur die Blendenplatten 8, 8a sichtbar sind.

Die Primärstrahlenblende 3 weist noch eine Irisblende 9 auf, die durch Keile die Ecken des durch die Rechteckblende 4 eingeblendeten Feldes abdeckt und dieses Feld demgemäß an den Eingangsleuchtschirm eines Röntgenbildverstärkers anpaßt. Ferner ist in zwei parallelen Ebenen ein Blendenplattenpaar 10, 20 vorgesehen, dessen Blendenplatten 10, 20 im Bereich der das Röntgenstrahlenbündel 2 begrenzenden Kanten keilförmig verlaufen, so daß eine Teiltransparenz für die Röntgenstrahlung gegeben ist. Dadurch ist das Heranführen von Instrumenten an das Untersuchungsobjekt im Röntgenbild sichtbar, ohne daß diagnostisch nicht relevante Bildbereiche überstrahlt werden. Schließlich ist ein Lichtvisier mit einer Lichtquelle 12 und einem für Röntgenstrahlen transparenten Spiegel 13 zum Sichtbarmachen des eingeblendeten Feldes auf dem Untersuchungsobjekt vorgesehen.

Die Blendenplattenpaare 5, 6 bzw. 5a, 6a müssen in ihren Ebenen senkrecht zum Zentralstrahl 7 so verstellt werden, daß sich die Blendenplatten 5, 5a; 6, 6a jeder Blendenseite in jeder Blendenstellung überlappen.

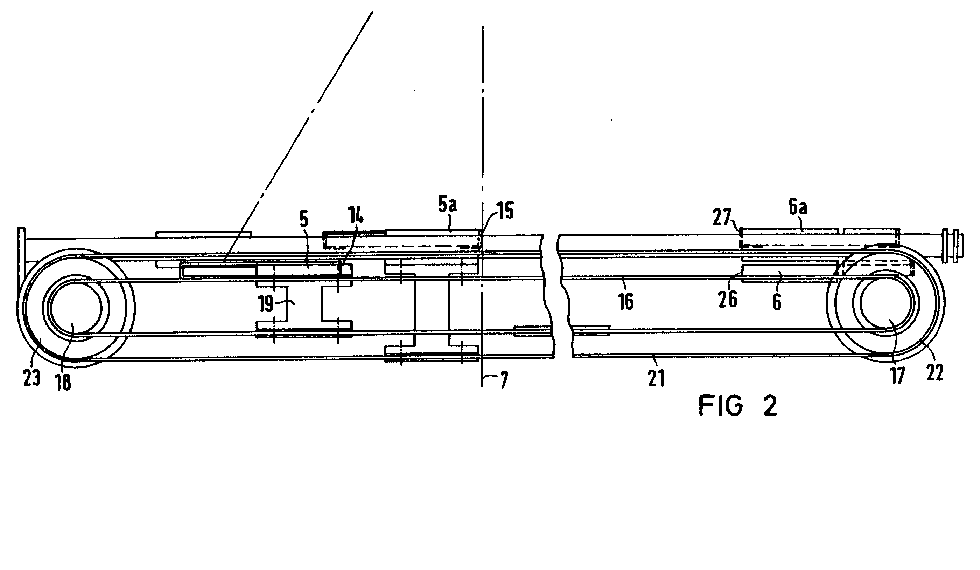

Hierzu sind die Blendenplatten 5 und 6 gemäß Figur 2 an einem Zahnriemen 16 befestigt, der um zwei Räder 17, 18 geführt ist. Damit sich die Blendenplatten 5, 6 gegenläufig zueinander bewegen, ist die Blendenplatte 5 dabei am unteren Teil des Zahnriemens 16 mit Hilfe eines Zwischenstückes 19 befestigt. Die Blendenplatten 5a, 6a sind an einem zweiten Zahnriemen 21 analog dazu befestigt, der um Räder 22, 23 geführt ist. Die Räder 17, 22 und 18, 23 sitzen auf derselben Achse und haben unterschiedliche Durchmesser.

Aufgrund des kleineren Durchmessers der Räder 17, 18 legen die Blendenplatten 5, 6 bei einer Drehung der Räder um einen bestimmten Winkel einen kleineren Weg zurück als die Blendenplatten 5a, 6a. Die Durchmesser der Räder 17, 18, 22, 23 sind so aufeinander abgestimmt, daß der Stellweg für das fokusnähere Blendenpaar 5a, 6a um soviel größer als der Stellweg für das fokusfernere Blendenplattenpaar 5, 6 ist, daß sich die parallelen Blendenplatten 5, 5a; 6, 6a jeder Blendenseite in jeder Blendenstellung überlappen.

Die Mittel zur Verstellung der senkrecht zu den Blendenplatten 5, 5a, 6, 6a verstellbaren Blendenplatten, von denen in der Figur 1 nur die Blendenplatten 8, 8a dargestellt sind, entsprechen den in der Figur 2 dargestellten Stellmitteln.

In der Figur 2 sind für die linke Hälfte der Blende die parallelen Blendenplatten 5, 5a in derjenigen Stellung gezeichnet, in der die Blende vollständig geschlossen ist. Die das Röntgenstrahlenbündel 2 begrenzende Kante 15 der Blendenplatte 5a ist dabei bis an den Zentralstrahl 7 herangeführt, während die rechte Kante 14 der Blendenplatte 5 unter der Blendenplatte 5a liegt. Die Blendenplatten 5, 5a überlappen sich demgemäß so weit, daß die Blende vollständig geschlossen ist. Dabei ist zu berücksichtigen, daß dabei die Blendenplatten 6, 6a eine spiegelbildliche Stellung einnehmen, in der die Kante 27 der Blendenplatte 6a ebenfalls am Zentralstrahl 7 und die Kante 26 der Blendenplatte 6 unter der Blendenplatte 6a liegt.

Die Blendenplatten 6, 6a sind in der Figur 2 in derjenigen Stellung gezeichnet, in der die Blende vollständig geöffnet ist. Dabei liegen die Kanten 26, 27 praktisch untereinander. Der Stellweg für die Blendenplatten 5, 6 ist dabei aufgrund der gewählten Untersetzung jeweils halb so groß wie der Stellweg für die Blendenplatten 5a, 6a. Aufgrund der Ausbildung der Blende mit zwei parallelen Blendenplatten 5, 5a; 6, 6a für jede Blendenseite kommt man mit schmalen Blendenplatten aus. Der Platzbedarf für die Blende ist demgemäß gering.

**Patentanspruch**

1. Primärstrahlenblende für Röntgendiagnostikgeräte mit mindestens zwei in parallelen Ebenen parallel zueinanderliegenden Blendenplattenpaaren (5, 6; 5a, 6a), deren Blendenplatten (5, 6; 5a, 6a) gegenläufig zueinander bewegbar sind, wobei die Blendenplatten (5, 6; 5a, 6a) jedes Paares an einem senkrecht zum Zentralstrahl (7) des Röntgenstrahlenbündels (2) verstellbaren, um Räder (17, 18, 22, 23) zu beiden Seiten des Zentralstrahles (7) geführten Zug befestigt sind, so daß der Stellweg für das fokusnähere Blendenplattenpaar (5a, 6a) um soviel größer als der Stellweg für das fokusferne Blendenplattenpaar (5, 6) ist, daß sich die parallelen Blendenplatten (5, 5a; 6, 6a) jeder Blendenseite in jeder Blendenstellung überlappen, dadurch gekennzeichnet, daß zu beiden Seiten des Zentralstrahles (7) jeweils zwei Räder mit unterschiedlichem Durchmesser und gemeinsamer Achse drehbar gelagert sind, wobei jedes Radpaar (17, 18, 22, 23) mit gleichem Durchmesser einen Zug für die Blendenplattenpaare (5, 6; 5a, 6a) trägt.

**Claim**

1. Primary radiation screen for X-ray diagnostic apparatus, having at least two pairs of screen plates (5, 6; 5a, 6a), lying parallel relative to each other and in parallel planes, the screen plates (5, 6; 5a, 6a) of which are movable in opposite directions to each other, the screen plates (5, 6; 5a, 6a) of each pair being mounted to a traction means, which means is adjustable perpendicularly to the central ray (7) of the beam of X-rays (2), and is guided around wheels (17, 18, 22, 23) at both sides of the central ray (7), so that the adjustment path for the pair of near focus screen plates (5a, 6a) is longer than the adjustment path for the pair of far focus screen plates (5, 6) by an amount such that the parallel screen plates (5, 5a; 6, 6a) on each side of the screen overlap in each screen position, characterised in that at each of both sides of the central ray (7) there are rotatably mounted about a common axis two wheels with different diameters, each pair of wheels (17, 18, 22, 23) with the same diameter carrying a traction means for the pairs of screen plates (5, 6; 5a, 6a).

**Revendication**

1. Diaphragme pour le rayonnement primaire pour des appareils de radiodiagnostic, comportant au moins un couple (5, 6; 5a, 6a) de plaques collimatrices, qui sont disposés parallèlement entre eux dans deux plans parallèles et dont les plaques collimatrices (5, 6; 5a, 6a) peuvent être déplacées en sens inverse les unes des autres, les plaques collimatrices (5, 6; 5a, 6a) de chaque couple étant fixées à un câble d'actionnement pouvant être déplacé perpendiculairement par rapport au rayon central (7) du faisceau (2) de rayons X et circulent sur des galets (17, 18, 22, 23) des deux côtés du rayon central (7), de sorte que la voie de réglage du couple (5a, 6a) de plaques collimatrices, qui est le plus proche du foyer, est supérieur à la course de réglage du couple (5, 6) de plaques collimatrices, qui est éloigné du foyer, au point que les plaques collimatrices parallèles (5, 5a; 6, 6a) de chaque côté du diaphragme se chevauchent dans toutes les positions de ce dernier, caractérisé par le fait que respectivement deux galets possédant des diamètres différents et un axe commun sont montés de façon à pouvoir tourner des deux côtés du rayon central (7), chaque couple de galets (17, 18, 22, 23) de même diamètre portant un câble d'actionnement pour les couples (5, 6; 5a, 6a) de plaques collimatrices.

FIG 1

FIG 2